# EUROPEAN PATENT APPLICATION

(11) **EP 2 529 677 A1**
(43) Date of publication of application: **05.12.2012**
(21) Application number: 11168095.5
(22) Date of filing: 30.05.2011
(51) Int. Cl.: A61B 17/15, A61B 17/17

(54) **Patient-specific surgical guiding tools, methods for manufacture**

(71) Applicant: Materialise NV, 3001 Leuven (BE)
(72) Inventor: Premanathan, Ayishwariya, 3000 Leuven (BE); Keppler, Louis James, 3000 Leuven (BE); Mohd Ismail, Mohd Azman, 52100 Kuala Lumpur (MY)
(74) Representative: Paemen, Liesbet R.J.

(57) **Abstract**

The present invention relates to patient-specific surgical guiding tools which are of use for guiding a surgical instrument during bone surgery. The patient-specific surgical guiding tools of the present invention provide for a stable and accurate guidance of a surgical instrument and comprise a one-piece longitudinally extending surface structure, comprising one or more patient-specific areas and at least three clamp members adapted to clamp on the bone. The present invention further provides methods for manufacturing the patient-specific surgical guiding tools of the invention and uses of the tools for placement onto a bone.

## Description

### FIELD OF THE INVENTION

The present invention relates to patient-specific surgical guiding tools which are of use for guiding a surgical instrument during bone surgery. The present invention further relates to methods for manufacturing these tools and specific methods for using the surgical guiding tools of the invention in bone surgery.

### BACKGROUND

Various surgical guides exist, which the surgeon can use to accurately transfer a pre-operative plan into the operating room with wide applications in orthopaedic surgery. These guides may guide a surgical instrument, such as cutting or drilling instrument, along a pre-defined cutting or drilling path, respectively. For a satisfactory outcome of the surgical procedure, it is essential that the surgical instrument is guided along the correct path.

US 4,860,735 discloses a drill alignment apparatus which is mounted on top of a drill handle and comprises an alignment rod and a clamp apparatus for fixing onto the bone. By placing the clamp on the bone, the drill handle can be moved only in the direction of the alignment rod. US 5,312,409 discloses a drill alignment guide, which comprises an adjustable clamp assembly for clamping onto the bone connected by an L-shaped bar to a drill guide. Upon clamping of the device on the bone, the drill guide is positioned on the bone head. US 4,896,663 discloses a femoral drill jig comprising a clamping mechanism which clamps on to the femoral neck and a head cone which comprising a guide bushing for guiding a drill into the femur head. Similarly, EP1588688 discloses an alignment guide for use in femoral surgery with either an arm or a moveable c-ring for fixing around the neck of the femur.

The above cited references describe generic, adjustable devices that consist of a guiding element, elements that can be positioned to clasp around a part of the bone, and a structure connecting the two. These devices are often unstable as they do not fit perfectly on the patient's bone and in some cases inaccurate because of the great distance between the supporting anatomy and the planned point of entry of the surgical instrument.

In addition, the prior art devices such as those referred to above are all quite bulky, mainly due to the presence of extended support arms and thus require the availability of a large surgical window.

Accordingly, there is a need for surgical guides, which do not require excessive image analysis and are not bulky but nevertheless are stable and provide the ability to accurately and efficiently guide a surgical instrument into or onto a patient's bone.

### SUMMARY OF THE INVENTION

The present invention relates to patient-specific surgical guiding tools which provide stable and accurate guidance of the surgical instrument. Moreover, as the tools ensure a direct fit in the desired position, no alignment is necessary, which saves time during placement.

In a first aspect, the present invention provides a patient-specific surgical guiding tool for a bone comprising (i) a one-piece longitudinally extending surface structure, comprising one or more patient-specific areas and at least three clamp members adapted to clamp on the bone, and (ii) at least one guiding component for guiding a surgical instrument connected to the surface structure.

More specifically, the present invention provides a patient-specific surgical guiding tool for a bone comprising:
(i) a one-piece longitudinally extending surface structure, comprising one or more patient-specific areas and at least three clamp members adapted to clamp on the bone, wherein
   - each of the at least three clamp members is formed by an extension of the surface structure to a contact point, whereby each of the contact points are located at different cross-sections of the longitudinal axis (x) of the surface structure, and
   - the at least three clamp members ensure that the surface structure spans the contour of the bone over at least 180° when the guiding tool is placed on the bone;
   the surgical guiding tool further comprising
(ii) at least one guiding component for guiding a surgical instrument connected to the surface structure.

In particular embodiments of the invention, the guiding tools are further characterized in that each of the at least three contact points corresponds to a patient-specific area of the surface structure.

In particular embodiments, the surface structure contains at least one weakened area having a thickness which is between 1/2 and 1/5 of the average thickness of the remainder of the surface structure. In certain embodiments, the minimum thickness of the at least one weakened area is about 2 mm. In further particular embodiments of the invention, the at least one weakened area is not located at one of the one or more patient-specific areas of the surface structure.

In particular embodiments of the surgical guiding tools described herein, the surface structure is completely patient-specific, and specifically fits with the anatomical surface of the bone.

In further particular embodiments of the surgical guiding tools of the invention, the at least one guiding component is a cutting slot or surface or a drill guide element.

In particular embodiments, the surgical guiding tools of the invention comprise at least one handle structure that protrudes from the outer surface of the surgical guiding tool. In more particular embodiments, the surgical guiding tools comprise at least two of said handle structures.

In a further aspect, the present invention provides methods for manufacturing the patient-specific surgical guiding tools of the invention.

More particularly, the present invention provides methods for manufacturing patient-specific surgical guiding tools of the invention for an intervention with a surgical tool on a bone, the methods comprising:
(a) obtaining an image of the bone,
(b) identifying and selecting one or more parts of the bone, which contain one or more patient-specific anatomical features,
(c) determining the appropriate position of the guiding component with regard to the bone, based on the pre-operative planning of the desired path of the surgical tool in the bone;
(d) designing the surgical guiding tool comprising a surface structure based on the information obtained in (a)-(c) wherein in the surgical guiding tool:
   (i) the position of three contact points of the surface structure ensures that the surface structure spans the contour of the bone over at least 180°;
   (ii) the location of one or more patient-specific areas in the surface structure ensures an optimal and specific fit with the anatomical surface of the bone; and
   (iii) the location of the guiding component on the surface structure ensures that, upon placement of the guiding tool on the bone, the guiding component is positioned as determined in step (c), and
(e) manufacturing the surgical guiding tool based thereon.

In particular embodiments, the devices of the present invention are partially or completely made by additive manufacturing. This not only facilitates making the one-piece surface structure but further allows the integration of patient-specific components which increase the accuracy of the guiding tools. Indeed, the guiding tools of the prior art are typically standard tools which can at most be adjusted mechanically to fit every patient, but do not ensure the patient-specific fit and accuracy.

In certain embodiments of the methods for manufacturing the patient-specific surgical guiding tools of the invention, step (d) of designing the surgical guiding tool further includes a substep (iv) of introducing areas of reduced thickness in the surface structure to increase flexibility thereof, such as for example by (but not limited to) determining which areas of the surface structure are displaced or distorted maximally upon placement of the guiding tool on the bone and adjusting the thickness or the shape of the areas that are displaced or distorted maximally upon placement of the guiding tool on the bone, thereby modulating the strength of the clamping force of the surface structure.

In yet a further aspect, the present invention relates to methods for using the tools of the present invention, more particularly methods for placing the guiding tools of the invention on a bone, and most particularly, methods for introducing a surgical instrument into a bone, which comprise placing the tools of the invention onto the bone.

According to this aspect, the present invention provides methods for introducing a surgical instrument into a bone, which methods comprise:
(a) securing a surgical guiding tool according to the present invention onto the bone, wherein the surgical guiding tool is characterized in that
   - it comprises a guiding component designed to guide the surgical instrument in a predefined trajectory;
   - it comprises a longitudinally extending surface structure, comprising at least three clamp members adapted to clamp on the bone, wherein each of the at least three clamp members is formed by an extension of the surface structure to a contact point, whereby the contact points are located at different cross-sections of the longitudinal axis (x) of the surface structure, and ensure the spanning of the contour of the bone over at least 180°, and
   - the surface structure comprises one or more patient-specific areas which are completely complementary and specifically fit to the anatomical surface of the bone,
   and the securing step comprises:
   - positioning the surgical guiding tool such that the one or more patient-specific areas are aligned with regard to their complementary anatomical surfaces on the bone,
   - clamping the surgical guiding tool over the bone,
   - if necessary, slightly rotating the surgical guiding tool to align the one or more patient-specific areas with the bone such that they lock into their respective complementary anatomical surface of the bone; and
(c) introducing the surgical instrument through the guiding component of the surgical guiding tool.

More particularly, the methods described hereinabove are methods for transferring a pre-planned intervention on or in the bone to surgery.

### BRIEF DESCRIPTION OF THE FIGURES

The following description of the figures of specific embodiments of the invention is merely exemplary in nature and is not intended to limit the present teachings, their application or uses. Throughout the drawings, corresponding reference numerals indicate like or corresponding parts and features.

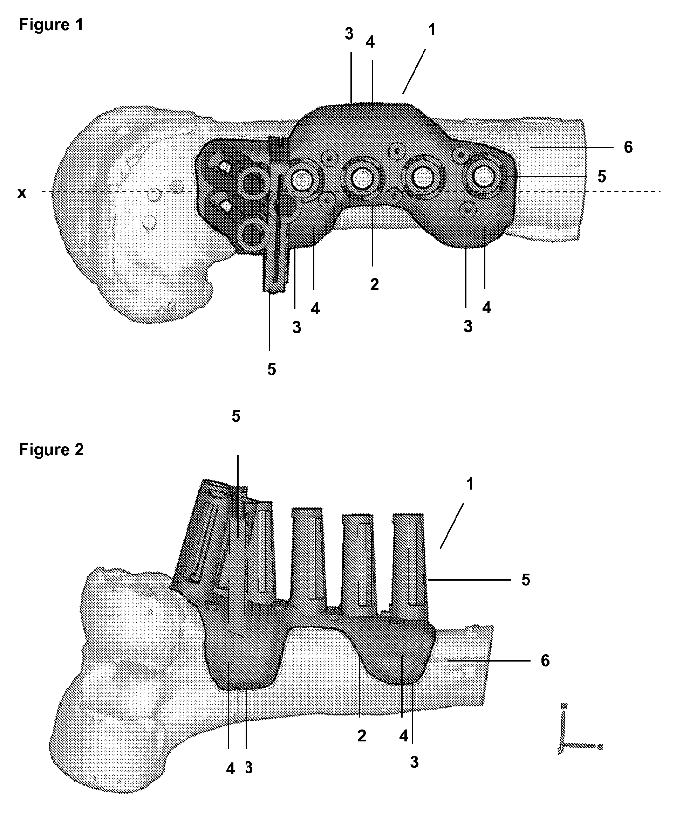

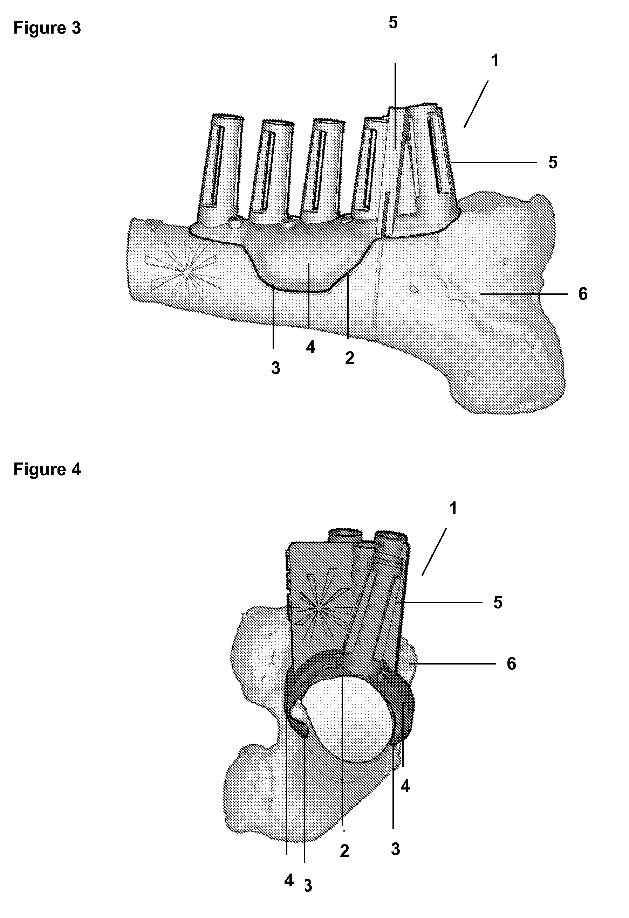

**Figures 1 to 4** Top view (Figure 1), left lateral view (Figure 2), right lateral view (Figure 3) and cross-sectional or longitudinal view (Figure 4) of a patient-specific surgical guiding tool (1) clamped onto the shaft of a bone (6) according to particular embodiments of the invention. The one-piece longitudinally extending surface structure (2), comprising one or more patient-specific areas, comprises at least three clamp members (3) adapted to clamp onto the bone (6). Each of the three clamp members (3) is formed by an extension of the surface structure to a contact point (4), whereby the contact points (4) are located at different cross-sections of the longitudinal axis (x) of the surface structure. The at least three clamp members (3) ensure that the surface structure (2) spans the contour of the bone (6) over at least 180° when the guiding tool is place on the bone (6). The surgical guiding tool (1) further comprises at least one guiding component (5) for guiding a surgical instrument connected to said surface structure (3).

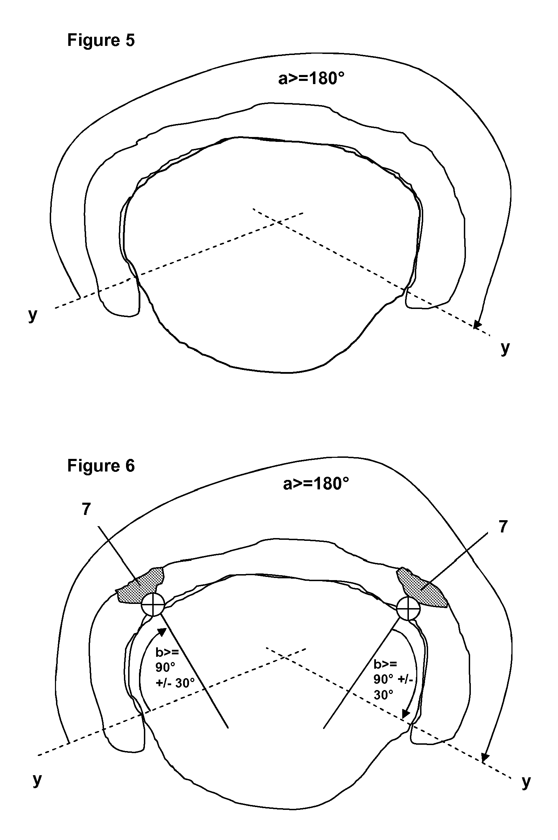

**Figure 5** Schematic cross-sectional view of a patient-specific surgical guiding tool (1) clamped onto the shaft of a bone (6) according to particular embodiments of the invention. The at least three alternating clamp members span the bone over an angle a of at least 180°, more particularly between 180° and 220°. An overhang of the ends of each of the clamping members is created when the guiding tool is placed on the bone thereby forming an "undercut". When the angular difference between the axis (y) perpendicular to the bone engagement surface in one contact point and the axis (y) perpendicular to the bone engagement surface in another contact point is larger than 180°, those two contact points form an undercut relative to the bone that is enclosed. The clamp members form a clamping or snapping mechanism, enclosing the bone by at least 180°. It is however clear from the present disclosure that the different clamp members extending into the different contact points are not located at the same cross-sectional position of the guiding tool with regard to the central longitudinal axis (x) of the tool. This is illustrated in Figures 1 to 4.

**Figure 6** Schematic cross-sectional view of a patient-specific surgical guiding tool (1) clamped onto the shaft of a bone (6) according to particular embodiments of the invention. The at least three alternating clamp members span the bone over an angle a of at least 180°, more particularly between 180° and 220°. The desired flexibility of the surface structure of the guiding tools according to these embodiments can be obtained by introducing weakened areas (7) in the surface structure allowing bending of the surface structure. In particular embodiments, at least one weakened area (7) has a thickness, which is between 1/2 and 1/5 of the average thickness of the remainder of the surface structure. In certain embodiments, the minimum thickness of the one or more weakened areas is about 2 mm, to maintain stability of the structure. In particular embodiments, the weakened areas are positioned in such a way that the angle b between the normal (y) of at least one of the at least three contact points to the bone anatomy engagement surface of the surface structure at that contact point and the normal to that at least one weakened area is about 90°+/- 30°.

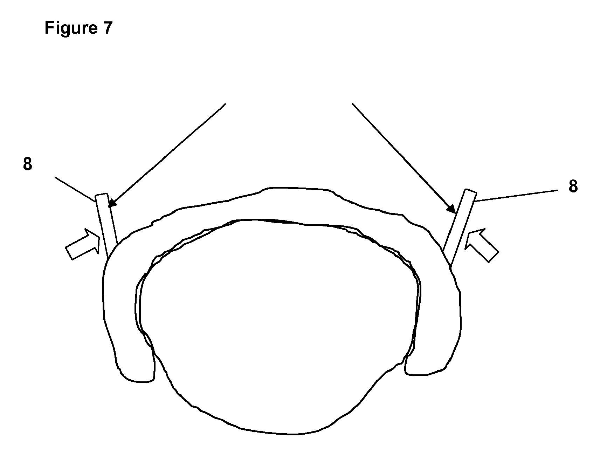

**Figure 7** Schematic cross-sectional view of a patient-specific surgical guiding tool (1) clamped onto the shaft of a bone (6) according to particular embodiments of the invention. In these particular embodiments, the surgical guiding tools of the invention comprise at least one handle structure (8) that protrudes from the outer surface of the surgical guiding tool. Such one or more handle structures may allow an easy manipulation of the guiding tools of the present invention, in particular for opening the guiding tools of the present invention and placing or removing these over or from the bone shaft. Such a handle thus may function as a lever. A handle structure comprised in the guiding tools of the present invention may function for example by means of a clothes peg mechanism, whereby if two or more handle structures are forced together the clamp members of the guiding tool are opened, thereby allowing an easier placement or removal of the guiding tool over or from the bone shaft. Furthermore, such handle structures can serve as an intuitive place for the surgeon to hold the guiding tool.

In the figures, the following numbering is used:
1 - Patient-specific surgical guiding tool
2 - Surface structure
3 - Clamp member
4 - Contact point
5 - Guiding component
6 - Bone
7 - Weakened area
8 - Handle structure

### DETAILED DESCRIPTION

The present invention will be described with respect to particular embodiments but the invention is not limited thereto but only by the claims. Any reference signs in the claims shall not be construed as limiting the scope thereof.

As used herein, the singular forms "a", "an", and "the" include both singular and plural referents unless the context clearly dictates otherwise.

The terms "comprising", "comprises" and "comprised of" as used herein are synonymous with "including", "includes" or "containing", "contains", and are inclusive or open-ended and do not exclude additional, non-recited members, elements or method steps. The terms "comprising", "comprises" and "comprised of" also includes embodiments "consisting of" the listed elements or steps.

Furthermore, the terms first, second, third and the like in the description and in the claims, are used for distinguishing between similar elements and not necessarily for describing a sequential or chronological order, unless specified. It is to be understood that the terms so used are interchangeable under appropriate circumstances and that the embodiments of the invention described herein are capable of operation in other sequences than described or illustrated herein.

The term "about" as used herein when referring to a measurable value such as a parameter, an amount, a temporal duration, and the like, is meant to encompass variations of +/-10% or less, preferably +/-5% or less, more preferably +/-1% or less, and still more preferably +/-0.1% or less of and from the specified value, insofar such variations are appropriate to perform in the disclosed invention. It is to be understood that the value to which the modifier "about" refers is itself also specifically, and preferably, disclosed.

The recitation of numerical ranges by endpoints includes all numbers and fractions subsumed within the respective ranges, as well as the recited endpoints.

All documents cited in the present specification are hereby incorporated by reference in their entirety.

Unless otherwise defined, all terms used in disclosing the invention, including technical and scientific terms, have the meaning as commonly understood by one of ordinary skill in the art to which this invention belongs. By means of further guidance, definitions for the terms used in the description are included to better appreciate the teaching of the present invention. The terms or definitions used herein are provided solely to aid in the understanding of the invention.

Reference throughout this specification to "one embodiment" or "an embodiment" means that a particular feature, structure or characteristic described in connection with the embodiment is included in at least one embodiment of the present invention. Thus, appearances of the phrases "in one embodiment" or "in an embodiment" in various places throughout this specification are not necessarily all referring to the same embodiment, but may. Furthermore, the particular features, structures or characteristics may be combined in any suitable manner, as would be apparent to a person skilled in the art from this disclosure, in one or more embodiments. Furthermore, while some embodiments described herein include some but not other features included in other embodiments, combinations of features of different embodiments are meant to be within the scope of the invention, and form different embodiments, as would be understood by those in the art. For example, in the following claims, any of the claimed embodiments can be used in any combination.

The present invention provides patient-specific surgical guiding tools which allow accurate and stable introduction of a surgical instrument into the bone. More particularly, the invention provides medical-image-based patient-specific surgical guiding tools providing the ability to accurately insert a surgical instrument into the patient's bone according to a predefined planning.

The term "patient-specific tool" as used herein refers to the surgical, devices, tools, or guides as described herein, which are designed starting from an individual patient's anatomy to provide patient-specific devices, tools, or guides having a custom fit or functioning in a unique, customized manner for a particular individual patient. The use of patient-specific devices, tools, or guides allows for improved or optimized surgical interventions, orthopaedic structures and/or kinematics for the patient. Similar benefits are obtained when such patient-specific devices are used in combination with standard implants, tools, devices, surgical procedures, and/or other methods.

Accordingly, in the context of the present invention, the term "patient-specific area" is used to describe an area that is mates with and/or is complementary with at least part of the patient's anatomy.

The surgical guiding tools according to the present invention at least comprise a one-piece longitudinally extending surface structure, for fitting onto the bone, and at least one guiding component for guiding a surgical instrument, which is connected to the surface structure. The one-piece longitudinally extending surface structure of the surgical guiding tools of the invention comprises, on the surface which fits onto the bone, one or more patient-specific areas and further comprises or extends into at least three clamp members adapted to clamp the bone.

The different components of the guiding tools according to the present invention are described more in detail hereafter. Unless specified (e.g. in the context of placement of the tools of the invention), reference to the position of the tool with regard to the bone is intended to refer to the position of the surgical guiding tool, when the tool has been placed onto the bone.

According to the present invention, optimal stability of the surgical guiding tools of the invention and thus accuracy of a surgical intervention using these tools is ensured by the presence of a surface structure that comprises at least three clamp members, each of which are formed by an extension of said surface structure to a contact point, whereby the contact points are located at different cross-sections of the longitudinal axis (x) of said surface structure. Thus, the three or more clamp members grip the bone at different positions along the longitudinal axis (x) of the surface structure. More particularly, the clamp members alternate and extend over the bone in opposite directions. However, the clamp members together ensure that the surface structure spans the contour of the bone over at least 180° when the guiding tool is placed on the bone. The devices of the present invention are further optionally provided with one or more patient-specific areas, which ensure a specific fit of the tool in a predetermined position onto one or more anatomical regions of the bone.

In the present description of the guiding tools of the invention, reference will be made to angles to define the extent to which the guiding tool structures span the contour of the bone. Indeed, the guiding tool structures contain a semi-cylindrical cavity formed by the surface structure and its extending clamp members for passage of the bone. It will be understood to the skilled person, that in general, while a difference of 10° on these angles may have a functional impact on the guiding tool, limited variations of the angles around the indicated values will not significantly impact the function of the structures of the guiding tool and can be considered as obvious alternatives of the values provided. Accordingly, where referring to an angle in the context of the present invention, unless specified, typically variations of +/- 5° should be considered as equally envisaged. Similarly, in some embodiments variations of +10° or -10° may also be envisaged. At the same time, it will be understood by the skilled person that, where reference is made to an angle of more or less than 180°, the cut-off is effectively 180° as this will impact the functionality of the relevant structure of the tool.

The at least three alternating clamp members span the bone over at least 180°, more particularly over at least 200° and preferably over between 180° and 300°, more particularly between 180° and 220°. An overhang of the ends of each of the clamping members is created when the guiding tool is placed on the bone thereby forming an "undercut". Thus, the surface structure extending into the clamp members typically has a cylindrical shape, with openings between the clamp members. The resilience/flexibility of the material from which the surface structure is made allows the user to exert a force such that the surface structure is slightly bent open to allow positioning onto the bone. The presence of at least three alternating clamp members spanning the bone over at least 180° ensures stability of the guiding tools when positioned on the bone. Indeed, the surgical guiding tools of the invention typically clamp and fit over at least part of the bone, such that the one or more guiding components connected thereto ensure accurate and stable guidance of a surgical instrument into the bone.

The extent to which the surface structure spans the bone is defined herein by referring to contact points. While the surface structure will of course be in contact with the bone when positioned thereon at multiple locations, the contact points referred to herein are those which define the extremities of the clamp members of the surface structure and thus determine the extent to which the surface spans the contour of the bone. Thus, according to the present invention, each of the at least three clamp members of the surface structure is formed by an extension of the surface structure to a defined contact point. Accordingly, the inner part of the surface structure (i.e. the area of the surface structure which faces the bone upon placement thereon) of the surgical guiding tools of the invention when placed on the bone, extend over the surface of the bone to at least three contact points, each corresponding to one of the at least three clamp members. More particularly, it is envisaged that the surface of the bone which is covered by the device of the present invention between the three contact points, extend over at least 180° of the circumference of the bone. Most particularly, the surface structure comprises three clamp members, whereby the surface of the bone which is covered between one contact point in one member and the two other contact points of the two other clamp members extends in both cases over 180°.

The at least three contact points each of which are comprised in one of the clamp members of the tools of the invention are located at different cross-sections of the longitudinal axis (x) of the surface structure, and thus of the longitudinal axis of the bone shaft when the tool is placed thereon. Indeed, the contact points, and thus the clamping members in which these are comprised, are located at different locations of the surface structure along its longitudinal axis (x). More particularly, the at least three clamping members alternately extend over the bone to their contact points in opposing directions. This implies that, viewed in cross-section of the device, one member extends in one direction over the bone (e.g. clockwise), while the two other members extend in the opposing direction (e.g. counter-clockwise), whereby the members extending in opposing direction alternate with respect to each other.

The extent to which the surface structure comprising at least three clamp members spans the bone contour over at least 180°, can also be expressed by referring to the normals in those contact points, which is the normal of the inner surface of the clamp member in that contact point. According to the present invention, the surface structure extends over the bone over the angle between two of these three contact points. The invention further provides that the device comprises at least three clamp members, each extending to a different contact point. It is envisaged that for two pairs formed by said at least three contact points, i.e. for two different combinations of two of the at least three contact points, the angle a that is formed by the normals to the bone anatomy engagement surface of the surface structure at those two contact points, is larger than 180°. Indeed, when the angular difference between the axis (y) perpendicular to the bone engagement surface in one contact point and the axis (y) perpendicular to the bone engagement surface in another contact point is larger than 180°, those two contact points form an undercut relative to the bone that is enclosed. The present invention envisages devices wherein the clamping is ensured by the undercut formed by at least three clamp members, more particularly by each of the two pairs of opposing clamp members (one clamp member common to both pairs). The clamp members form a clamping or snapping mechanism, enclosing the bone by at least 180°. It is however clear from the present disclosure that the different clamp members extending into the different contact points are not located at the same cross-sectional position of the guiding tool with regard to the central longitudinal axis (x) of the tool. This is illustrated in Figures 1 to 4.

The one-piece longitudinally extending surface structure of the patient-specific surgical guiding tools of the invention may further comprise one or more patient-specific areas. A patient-specific area is an area on the inside of the surface structure (i.e. the surface intended to face the bone), which is complementary to the surface of the bone on which the tool is to be placed in a patient-specific way or contains features which specifically mate with features on the bone and thus "interlocks" with this surface. Typically, a patient-specific area is a surface that is fully complementary to a surface on the bone. In particular embodiments, this complementarity implies taking into account a clearance between the guide tool surface and the patient's bone of between 0.1-0.2 mm.

The one or more patient-specific areas are positioned on the inside of the surface structure such that they can be contacted with/positioned opposite to the corresponding surface of the bone upon placement of the guiding tool. When a patient-specific area of the surface structure is contacted with/positioned opposite to its corresponding complementary part of the surface of the bone, the two surfaces interlock, fixing the surface structure (and thus the guiding tool) into a predetermined position. This ensures optimal accuracy of placement of the guiding tool.

Typically, a patient-specific area is pre-selected based on suitable and unique anatomical features present on the bone. However it can be envisioned that, in particular embodiments, features are introduced into the patient's bone to allow the generation of a patient-specific area based thereon.

The size of the one or more patient-specific areas of the surface structure of the guiding tools of the present invention is not critical. Depending on the embodiment, they can occupy all or part of the inside of the surface structure.

The location of the one or more patient-specific areas on the surface structure is also not critical and may be spread out over one or more parts of the surface structure.

As indicated above, the surface structure of the guiding tools according to the present invention consists of one piece and typically extends longitudinally corresponding to at least part of the length of the bone for which it is envisaged. The Each of the at least three clamp members of the surface extends in a direction which is not parallel to the longitudinal axis (x) of the surface structure and thus not parallel to the longitudinal axis of the bone on which it is placed. In particular embodiments, each of the at least three clamp members extends in a direction which is substantially perpendicular to the longitudinal axis (x) of the surface structure and thus substantially perpendicular to the longitudinal axis of the bone on which it is placed.

As indicated above, the surface structure comprising the at least three clamp members has a certain flexibility such that it can autonomously clamp the bone. Such a resilience implies that during placement of the guiding tool onto the bone, each of the at least three clamp members is displaced such that the shaft of the bone can be introduced within a semi-cylindrical cavity formed by the surface structure and its extending clamp members. The extent of displacement of the at least three clamp members (and thus the flexibility of the guiding tool) that is needed to place the guiding tool onto the bone depends on the thickness of the shaft of the bone and the extent to or the angle by which the clamp members span the contour of the bone upon placement of the guiding tool thereon.

In particular embodiments of the invention, the guiding tools are further characterized in that the at least three contact points of the clamp members correspond to a patient-specific area of the surface structure. Indeed, in these embodiments the device not only contacts the bone in the contact points but specifically fit onto the bone in these contact points.

In further particular embodiments of the surgical guiding tools described herein, the inner part of the surface structure (which extends to and thus includes the inner part of the at least three clamp members) is completely patient-specific, and specifically fits with the anatomical surface of the bone.

The desired flexibility of the surface structure to allow positioning on the bone can be obtained in different ways. In particular embodiments, the surface structure contains areas which are weakened such that they allow bending of the surface structure. In more particular embodiments at least one weakened area having a thickness which is between 1/2 and 1/5 of the average thickness of the remainder of the surface structure. In these particular embodiments, the surface structure has a reduced thickness at certain locations, such that fulcrum-like points are created, which will alter the strength of the snapping force of the guiding tools of the invention. Accordingly, by introducing one or more "weakened areas" in the surface structure of the guiding tools of the invention, the flexibility of the guiding tools and thus the ease by which they can be snapped onto the bone can be modulated. In addition to the introduction of "weakened areas", the flexibility of the guiding tools may also be modulated by introducing breaks in the surface structure or locally replacing parts of the material of the surface structure by a different material having a different material strength.

In certain embodiments, the minimum thickness of the one or more weakened areas is about 2 mm, to maintain stability of the structure.

The at least one weakened area that is comprised in the surface structures of the guiding tools of the invention may be located anywhere on the surface structure. However, in particular embodiments, in order to optimally improve the flexibility of the guiding tools by means of the presence of one or more weakened areas, such weakened areas are positioned on the surface structure in those regions which require flexibility to allow bending of the clamping members upon positioning of the structure on the bone. Accordingly, in particular embodiments, the weakened areas are positioned in such a way that the angle b between the normal (y) of at least one of said at least three contact points to the bone anatomy engagement surface of the surface structure at that contact point and the normal to said at least one weakened area is about 90°+/- 30°. Accordingly, optimal improvement of flexibility or resilience of the guides will be obtained when the axis which runs perpendicular to the bone engagement surface of the surface structure in a particular contact point is more or less perpendicular to the axis that runs perpendicular to a weakened area of that surface.

In further particular embodiments of the invention, the at least one weakened area is not located at one of the one or more patient-specific areas of the surface structure. For example, where the patient-specific areas of the guiding tools of the invention are limited to the regions where the at least three contact points engage with the bone, the weakened areas are in certain embodiments not located in these patient-specific areas.

However, in those embodiments, where the surface structure of the guiding tools of the invention is completely or partially patient-specific and the patient-specific areas are not limited to the positions of the at least three contact points, the one or more weakened areas can be located in a patient-specific area of the surface structure.

In further particular embodiments, the surgical guiding tools of the invention comprise at least one handle structure that protrudes from the outer surface of the surgical guiding tool. Such one or more handle structures may allow an easy manipulation of the guiding tools of the present invention, in particular for opening the guiding tools of the present invention and placing or removing these over or from the bone shaft. Such a handle thus may function as a lever. A handle structure comprised in the guiding tools of the present invention may function for example by means of a mechanism similar to that of a clothes pin or peg, whereby if two or more handle structures are forced together the clamp members of the guiding tool are opened, thereby allowing an easier placement or removal of the guiding tool over or from the bone shaft. Thus in particular embodiments, such handles are placed tangentially on the surface structure. In particular embodiments the handles extend from the surface at the base of two opposing clamp members. Furthermore, such handle structures can serve as an intuitive place for the surgeon to hold the guiding tool.

The surgical guiding tools of the present invention further comprise at least one guiding component for guiding a surgical instrument into the bone of a patient. The guiding component contains at least one means of guiding an instrument such as but not limited to a drill, bur, saw, jig saw, lateral drill or any other cutting, milling or drilling instrument, the orientation and position of which corresponds to a pre-operative planning. The guiding component can optionally include safety stops to prevent a surgical instrument from advancing beyond a planned or determined depth into the bone.

Thus, where the surgical instrument is a drill or bur, the guiding component may comprise at least a cylindrical hole, the position and direction of which correspond to the pre-operatively planned drilling or burring trajectory. The diameter of the cylindrical hole will be at least the size of the diameter of the part of the surgical instrument, which is to be introduced in the bone.

In particular embodiments, where the surgical instrument is a saw, jig saw, mill or lateral drill, the guiding component may contain at least a (narrow) slot or flat surface, the position and orientation of which will correspond to the pre-operatively planned cutting plane or milling surface. The dimensions of the slot or surface will be such to allow passage or suitable guidance of the saw, jig saw, mill or lateral drill that is used.

Accordingly, in particular embodiments, the guiding component includes a cannula or channel through which at least part of a surgical instrument may be passed to engage the bone at the desired location.

Typically, the guiding component is positioned on the surface structure. The guiding component is positioned such that a surgical instrument which is passed through the guiding component can engage the bone at the desired location. The guiding component can be positioned in any direction with regard to the central axis of the bone as long as it provides access to a surgical instrument for reaching the bone at the desired location.

The central axis of the guiding component can be considered the axis of the direction of the planned trajectory of the surgical instrument. According to particular embodiments, the guiding component is positioned in the guiding tool such that the central axis of the guiding component forms an angle with the central axis of the bone upon placement of the surgical guiding tool on the bone.

In a further aspect, the present invention provides methods for manufacturing the patient-specific surgical guiding tools of the invention.

More particularly, the present invention provides methods for manufacturing patient-specific surgical guiding tools of the invention for an intervention with a surgical tool on a bone, the methods comprising:
(a) obtaining an image of the bone,
(b) identifying and selecting one or more parts of the bone, which contain one or more patient-specific anatomical features,
(c) determining the appropriate position of the guiding component with regard to the bone, based on the pre-operative planning of the desired path of the surgical tool in the bone;
(d) designing the surgical guiding tool comprising a surface structure based on the information obtained in (a)-(c) wherein in the surgical guiding tool:
   (i) the position of three contact points of the surface structure ensures that the surface structure spans the contour of the bone over at least 180°
   (ii) the location of one or more patient-specific areas in the surface structure ensures an optimal and specific fit with the anatomical surface of the bone; and
   (iii) the location of the guiding component on the surface structure ensures that, upon placement of the guiding tool on the bone, the guiding component is positioned as determined in step (c), and
(e) manufacturing the surgical guiding tool based thereon.

In particular embodiments, the devices of the present invention are partially or completely made by additive manufacturing. This allows the integration of patient-specific components which further increase the accuracy of the guiding tools. Indeed, the guiding tools of the prior art are typically standard tools which can at most be adjusted mechanically to fit every patient, but do not ensure the patient-specific fit and accuracy.

Accordingly, step (a) of the methods of manufacturing the tools according to the invention comprises obtaining an image of the bone. Digital patient-specific image information can be provided by any suitable means known in the art, such as for example a computer tomography (CT) scanner, a magnetic resonance imaging (MRI) scanner or an ultrasound scanner. A summary of medical imaging has been described in "Fundamentals of Medical imaging", by P. Suetens, Cambridge University Press, 2002. However, it is an advantage of the tools of the present invention that sufficient information can be obtained for designing the guiding tools of the invention based on either CT or MRI images.

Typically, step (a) of the methods described above comprises the step of (a1) obtaining a 2D dataset of the bone and (a2) reconstructing a 3d virtual bone model (or part thereof) from said 2D datasets. Indeed, the first step in pre-operative planning is the construction of a 3D virtual model of the bone (or a part thereof). This reconstruction starts with sending a patient to a radiologist for scanning, e.g. for a scan that generates medical volumetric data, such as a CT, MRI scan or the like. The output of the scan can be a stack of two-dimensional (2D) slices forming a 3D data set. The output of the scan can be digitally imported into a computer program and may be converted using algorithms known in the field image processing technology to produce a 3D computer model of a relevant bone. Preferably, a virtual 3D model is constructed from the dataset using a computer program such as Mimics™ as supplied by Materialise N.V., Leuven, Belgium. A more detailed description for making a perfected model is disclosed in U.S. Patent No. 5,768,134 entitled 'Method for making a perfected medical model on the basis of digital image information of a part of the body'.

Once the 3D volume of the bone (or a part thereof) is reconstructed, the surgeon (or person skilled in the art) can define the preferred position, orientation, depth and diameter of the bores and drill paths associated to them.

The methods for manufacturing the surgical guiding tools according to the invention further comprise the step of identifying and selecting at least one part of the bone which contains sufficient features such that it is patient-specific. Indeed, an important aspect of the guiding tools of the invention is the presence of one or more patient-specific areas in the surface structure of the tool, which interlock(s) with the corresponding region(s) of the bone upon placement of the tool thereon and ensure accurate positioning of the tool. As indicated above, the patient-specific regions of the bone may comprise specific anatomical features or may be modified to introduce specific features. The production of the one or more patient-specific areas for the surface structure requires detailed, geometrical patient-specific information in order to determine those surfaces of the bone that are suitable for this purpose.

The methods for manufacturing the surgical guiding tools according to the invention further comprise the step of determining the appropriate position of the guiding component with regard to the bone. In particular embodiments, this is done based on the pre-operative planning of the desired path of the surgical tool in the bone.

The orientation of the guiding component has to be such that the surgical instrument is guided in the predetermined direction. Pre-operative planning of the intervention by the treating physician makes it possible to determine the required path of the surgical instrument, and accordingly, the required orientation of the guiding component. The pre-operative planning of the surgical intervention is done using suitable dedicated software, based on suitable medical images (of which CT, MRI, are examples), taking into account factors like bone quality and proximity to nerve bundles/blood vessels or other anatomically sensitive objects. To plan and simulate the intervention, preoperative images are imported into a computer workstation running 3D software. These images are manipulated as 3D volumes. The result is a computer simulation of the intervention, which outputs a planning containing the information necessary for adapting the orientation of the guiding component.

A further step of the methods for manufacturing a guiding tool according to the invention comprises preparing a surgical guiding tool according to the present invention. As detailed hereinabove, such a surgical guiding tool comprises
(i) a one-piece longitudinally extending surface structure, comprising one or more patient-specific areas and at least three clamp members adapted to clamp on the bone, wherein each of the at least three clamp members
   - is formed by an extension of the surface structure to a contact point, whereby the contact points are located at different cross-sections of the longitudinal axis (x) of the surface structure, and
   - ensures that the surface structure spans the contour of the bone over at least 180° when the guiding tool is place on the bone; and
ii) at least one guiding component for guiding a surgical instrument connected to the surface structure.

In particular embodiments, the step of preparing a surgical guiding tool comprises designing the surgical guiding tool wherein:
(i) the position of three contact points of the surface structure ensures that the surface structure spans the contour of the bone over at least 180°,
(ii) the location of one or more patient-specific areas in the surface structure ensures an optimal and specific fit with the anatomical surface of the bone; and
(iii) the location of the guiding component on the surface structure ensures that, upon placement of the guiding tool on the bone, the guiding component is positioned as determined in step (c).

In particular embodiments, the step of preparing the surgical guiding tool implies producing and assembling the components of the surgical guiding tool according to the invention, i.e. producing a one-piece surface structure (as described herein) comprising one or more patient-specific areas and at least three clamp members and at least one guiding component (as described herein). In particular embodiments, where one or more of the components of the guiding tools of the present invention are made of one piece, the step of "assembling" may correspond to combining the relevant information for the combined manufacture of the pieces involved.

More particularly, the step of preparing the surgical guiding tool can involve positioning the surface structure and the guiding component such that the guiding tool fits onto the bone. The surface structure is positioned such that it allows interlocking of the patient-specific area(s) with the corresponding region(s) on the bone. In particular embodiments, the step of positioning the different elements can be performed as part of the design of the device.

In particular embodiments, the methods of the present invention comprise preparing a surface structure comprising one or more patient-specific areas based on the patient-specific parts of the bone identified as described above. The one or more patient-specific area(s) is(are) made by generating a surface which is complementary to a patient-specific surface of the bone of the patient. For converting a digital image information of a part of the body into a basic model, template or mould, of which at least a part perfectly shows the positive or negative form of at least a portion of the part of the body, any suitable technique known in the art can be used, such as for example the rapid prototyping technique as described herein.

In particular embodiments, the step of preparing the surgical guiding tool comprises the production of one or more elements by rapid prototyping. In further particular embodiments, the step of preparing the surgical guiding tool comprises the production of the surgical guiding tool as defined in the present invention, by rapid prototyping.

Rapid Prototyping and Manufacturing (RP&M) can be defined as a group of techniques used to quickly fabricate a scale model of an object typically using three-dimensional (3-D) computer aided design (CAD) data of the object. Currently, a multitude of Rapid Prototyping techniques is available, including stereo lithography (SLA), Selective Laser Sintering (SLS), Fused Deposition Modeling (FDM), foil-based techniques, etc.

A common feature of these techniques is that objects are typically built layer by layer. Stereo lithography, presently the most common RP&M technique, utilizes a vat of liquid photopolymer "resin" to build an object a layer at a time. On each layer, an electromagnetic ray, e.g. one or several laser beams which are computer-controlled, traces a specific pattern on the surface of the liquid resin that is defined by the two-dimensional cross-sections of the object to be formed. Exposure to the electromagnetic ray cures, or, solidifies the pattern traced on the resin and adheres it to the layer below. After a coat had been polymerized, the platform descends by a single layer thickness and a subsequent layer pattern is traced, adhering to the previous layer. A complete 3-D object is formed by this process.

Selective laser sintering (SLS) uses a high power laser or another focused heat source to sinter or weld small particles of plastic, metal, or ceramic powders into a mass representing the 3-dimensional object to be formed.

Fused deposition modeling (FDM) and related techniques make use of a temporary transition from a solid material to a liquid state, usually due to heating. The material is driven through an extrusion nozzle in a controlled way and deposited in the required place as described among others in U.S. Pat. No. 5.141.680.

Foil-based techniques fix coats to one another by means of gluing or photo polymerization or other techniques and cut the object from these coats or polymerize the object. Such a technique is described in U.S. Pat. No. 5.192.539.

Typically RP&M techniques start from a digital representation of the 3-D object to be formed. Generally, the digital is sliced into a series of cross-sectional layers which can be overlaid to form the object as a whole. The RP&M apparatus uses this data for building the object on a layer-by-layer basis. The cross-sectional data representing the layer data of the 3-D object may be generated using a computer system and computer aided design and manufacturing (CAD/CAM) software.

A selective laser sintering (SLS) apparatus is particularly preferred for the manufacture of the guiding template from a computer model. It should be understood however, that various types of rapid manufacturing and tooling may be used for accurately fabricating these surgical templates including, but not limited to, stereolithography (SLA), Fused Deposition Modeling (FDM) or milling.

The surgical guiding tools of the invention (or parts thereof) may be manufactured in different materials. Typically, only materials that are biocompatible (e.g. USP class VI compatible) with the human body are taken into account. Preferably the surgical template is formed from a heat-tolerable material allowing it to tolerate high-temperature sterilization. In the case SLS is used as a RP&M technique, the surgical template may be fabricated from a polyamide such as PA 2200 as supplied by EOS, Munich, Germany or any other material known by those skilled in the art may also be used.

In certain embodiments of the methods for manufacturing the patient-specific surgical guiding tools of the invention, step (d) of designing the surgical guiding tool further includes a substep (iv) of introducing areas of reduced thickness in the surface structure to increase flexibility thereof.

Such areas of reduced thickness are also referred to as weakened areas (as described herein) having a thickness which is between 1/2 and 1/5 of the average thickness of the remainder of the surface structure. Indeed, by introducing a reduced thickness at certain locations of the surface structure of the guiding tools of the invention, fulcrum-like points are created, which will alter the strength of the snapping force of the guiding tools of the invention. Accordingly, by introducing one or more "weakened areas" in the surface structure of the guiding tools of the invention, the flexibility of the guiding tools and thus the ease by which they can be snapped onto the bone can be modulated and optimized. The at least one weakened area that is comprised in the surface structures of the guiding tools of the invention may be located anywhere on the surface structure. However, in order to obtain a maximum improvement of flexibility or resilience of the guiding tools of the invention, weakened areas of reduced thickness can for example be introduced at certain pre-determined locations of the surface structure. These locations can be determined by identifying those areas of the surface structure that are displaced or distorted maximally upon placement of the guiding tool on the bone. Subsequently, the thickness or the shape of the areas that are displaced or distorted maximally upon placement of the guiding tool on the bone can be adjusted, thereby modulating the strength of the clamping force of the surface structure on the bone. In particular embodiments, in order to optimally improve the flexibility of the guiding tools by means of the presence of one or more weakened areas, such weakened areas are positioned on the surface structure in such a way that the angle b between the normal (y) of at least one of said at least three contact points to the bone anatomy engagement surface of the surface structure at that contact point and the normal to said at least one weakened area is about 90°+/- 30°. Accordingly, optimal improvement of flexibility or resilience of the guides will be obtained when the axis which runs perpendicular to the bone engagement surface of the surface structure in a particular contact point is more or less perpendicular to the axis that runs perpendicular to a weakened area of that surface.

In addition to the introduction of "weakened areas", the flexibility of the guiding tools may also be modulated by introducing breaks in the surface structure or locally replacing parts of the material of the surface structure by a different material having a different material strength.

In a further aspect, the present invention relates to methods for using the tools of the present invention, more particularly methods for placing the guiding tools of the invention on a bone, and most particularly, methods for introducing a surgical instrument into a bone, which comprise placing the tools of the invention onto the bone.

Accordingly, in this aspect, the present invention provides methods for introducing a surgical instrument into a bone, which methods comprise:
(a) securing a surgical guiding tool according to the present invention onto the bone, wherein
   the surgical guiding tool is characterized in that
   - it comprises a guiding component designed to guide the surgical instrument in a predefined trajectory;
   - it comprises a longitudinally extending surface structure, comprising at least three clamp members adapted to clamp on the bone, wherein each of the at least three clamp members is formed by an extension of the surface structure to a contact point, whereby the contact points are located at different cross-sections of the longitudinal axis (x) of the surface structure, and ensure the spanning of the contour of the bone over at least 180°,
   - the surface structure comprises one or more patient-specific areas which are completely complementary and specifically fit to the anatomical surface of the bone,
   and the securing step comprises:
   - positioning the surgical guiding tool such that the one or more patient-specific areas are aligned with regard to their complementary anatomical surfaces on the bone,
   - clamping the surgical guiding tool over the bone,
   - if necessary, slightly rotating the surgical guiding tool to align the one or more patient-specific areas with the bone such that they lock into their respective complementary anatomical surface of the bone; and
(c) introducing the surgical instrument through the guiding component of the surgical guiding tool.

More particularly, the methods described hereinabove are methods for transferring a pre-planned intervention on or in the bone to surgery.

Methods for producing the surgical guiding tool according to the invention are detailed hereinabove.

The methods for accurately positioning a surgical instrument onto bone and/or introducing a surgical instrument into a bone of the present invention further comprise the step of securing the surgical guiding tool on the bone.

More particularly, the step of securing the surgical guiding tool on the bone implies that the guiding tool is placed onto the patient's bone in such a manner so as to allow accurate and stable placement and optionally introduction of a surgical instrument onto/into the bone. This involves the positioning the surgical guiding tool such that the one or more patient-specific areas of the surface structure are aligned with regard to their complementary anatomical surfaces on the bone.

This implies that the guiding tool is placed or clamped onto or around the bone by moving the bone shaft into the semi-cylindrical cavity formed by the surface structure and its extending clamp members and by ensuring that the patient-specific areas of the surface structure are directly contacted with their corresponding anatomical regions of the bone upon placement. Typically, the guiding tool is moved towards the bone shaft such that the longitudinal axis (x) of the surface structure of the guiding tool and the longitudinal axis of the bone shaft are parallel to each other, and the direction of movement is perpendicular to these longitudinal axes.

In certain embodiments, an additional slight rotation movement of the device relative to the bone may be required to ensure a tight fit of the tool on the bone. Accordingly, in these embodiments of the methods of the invention, the securing step further comprises slightly rotating the surgical guiding tool to align the one or more patient-specific areas with the bone such that they lock into their respective complementary anatomical surface of the bone.

The methods for introducing a surgical instrument into a bone according to the invention further comprise, after securing the surgical guiding tool on the bone the step of introducing the surgical instrument through the guiding component. Thus, in the methods according to the present invention, the guiding component of the surgical guiding tools is positioned on a unique and stable position, specific for a patient and intended to be used for guiding a surgical instrument, according to a prior determined position, direction and/or path and/or depth. At least part of the surgical instrument is therefore introduced through a hole, a cannula, a channel or a slot or along a flat surface to engage the bone at the desired location.

It will be appreciated by persons skilled in the art that numerous variations and/or modifications may be made to the invention without departing from the spirit or the scope of the invention as broadly described. The above described embodiments are, therefore, to be considered in all respects as illustrative and not restrictive.

## Claims

1. A patient-specific surgical guiding tool for a bone comprising:
(i) a one-piece longitudinally extending surface structure, comprising one or more patient-specific areas and at least three clamp members adapted to clamp on the bone, wherein
- each of said at least three clamp members is formed by an extension of said surface structure to a contact point, whereby the contact points are located at different cross-sections of the longitudinal axis (x) of said surface structure, and
- said at least three clamp members ensure that the surface structure spans the contour of the bone over at least 180° when the guiding tool is placed on the bone;
and
(ii) at least one guiding component for guiding a surgical instrument connected to said surface structure.

2. The patient-specific surgical guiding tool according to claim 1, wherein each of said at least three contact points corresponds to a patient-specific area of the surface structure.

3. The patient-specific surgical guiding tool according to claims 1 or 2, wherein said surface structure contains at least one weakened area having a thickness which is between 1/2 and 1/5 of the average thickness of the remainder of said surface structure and the minimum thickness of the at least one weakened area is about 2 mm.

4. The patient-specific surgical guiding tool according to any of claims 1 to 3, wherein said at least one weakened area is not located at one of said one or more patient-specific areas of said surface structure.

5. The patient-specific surgical guiding tool according to any of claims 1 to 4, wherein said surface structure is completely patient-specific, and specifically fits with the anatomical surface of the bone.

6. The patient-specific surgical guiding tool according to any of claims 1 to 5, wherein one of said at least one guiding component is a cutting slot or surface.

7. The patient-specific surgical guiding tool according to any of claims 1 to 6, wherein one of said at least one guiding component is a drill guide element.

8. The patient specific surgical guiding tool according to any of the claim 1 to 7, comprising two handle structures that protrude from the outer surface of the surgical guiding tool.

9. A method for manufacturing a patient-specific surgical guiding tool for an intervention with a surgical tool on a bone according to any one of claims 1 to 8, said method comprising:
(a) obtaining an image of the bone,
(b) identifying and selecting one or more parts of the bone, which contain one or more patient-specific anatomical features,
(c) determining the appropriate position of the guiding component with regard to the bone, based on the pre-operative planning of the desired path of the surgical tool in the bone;
(d) designing the surgical guiding tool comprising a surface structure based on the information obtained in (a)-(c) wherein in said surgical guiding tool:
(i) the position of three contact points of said surface structure ensures that the surface structure spans the contour of the bone over at least 180°
(ii) the location of one or more patient-specific areas in the surface structure ensures an optimal and specific fit with the anatomical surface of the bone; and
(iii) the location of the guiding component on said surface structure ensures that, upon placement of the guiding tool on the bone, the guiding component is positioned as determined in step (c), and
(e) manufacturing the surgical guiding tool based thereon.

10. The method according to claim 9, wherein said step (d) of designing the surgical guiding tool further includes a substep (iv) of introducing areas of reduced thickness in said surface structure to increase flexibility thereof.

11. The method according to claim 10, wherein said substep (iv) comprises determining which areas of the surface structure are displaced or distorted maximally upon placement of the guiding tool on the bone and adjusting the thickness or the shape of said areas that are displaced or distorted maximally upon placement of the guiding tool on the bone, thereby modulating the strength of the clamping force of said surface structure.

12. A method for introducing a surgical instrument into a bone, which method comprises:
(a) securing a surgical guiding tool according to any one of claims 1 to 8 onto the bone, wherein
said surgical guiding tool is **characterized in that**
- it comprises a guiding component designed to guide said surgical instrument in a predefined trajectory;
- it comprises a longitudinally extending surface structure, comprising at least three clamp members adapted to clamp on the bone, wherein each of said at least three clamp members is formed by an extension of said surface structure to a contact point, whereby the contact points are located at different cross-sections of the longitudinal axis (x) of said surface structure, and ensure the spanning of the contour of the bone over at least 180°,
- said surface structure comprises one or more patient-specific areas which are completely complementary and specifically fit to the anatomical surface of the bone,
and said securing comprises:
- positioning the surgical guiding tool such that said one or more patient-specific areas are aligned with regard to their complementary anatomical surfaces on the bone,
- clamping the surgical guiding tool over the bone,
- if necessary, slightly rotating the surgical guiding tool to align said one or more patient-specific areas with the bone such that they lock into their respective complementary anatomical surface of the bone; and
(c) introducing the surgical instrument through the guiding component of said surgical guiding tool.
